# EUROPÄISCHE PATENTSCHRIFT

(11) **EP 0 983 468 B1**
(45) Veröffentlichungstag und Bekanntmachung des Hinweises auf die Patenterteilung: **05.11.2003**
(21) Anmeldenummer: 98932065.0
(22) Anmeldetag: 20.05.1998
(51) Int. Cl.: F16M 11/04

(54) **MIKROSKOPSTATIV, INSBESONDERE FÜR EIN OPERATIONSMIKROSKOP**
MICROSCOPE STAND, ESPECIALLY FOR AN OPERATION MICROSCOPE
STATIF POUR MICROSCOPE, EN PARTICULIER POUR MICROSCOPE D'OPERATIONS

(30) Priorität: 20.05.1997 CH 116397
(43) Veröffentlichungstag der Anmeldung: 08.03.2000
(73) Patentinhaber: Leica Mikroskopie Systeme AG, 9435 Heerbrugg (CH)
(72) Erfinder: METELSKI, Andreas, CH-8590 Romanshorn (CH); WÄGER, Karl-Heinz, A-6840 Götzis (AT)
(74) Vertreter: Reichert, Werner F., Dr.
(86) Internationale Anmeldenummer: EP9802964
(87) Internationale Veröffentlichungsnummer: WO98053244

(56) Entgegenhaltungen:
- EP-A- 0 476 551
- EP-A- 0 476 552
- EP-A- 0 628 290
- WO-A-97/13997
- WO-A-97/20166
- CH-A- 625 057
- DE-C- 4 214 858
- US-A- 2 810 819
- US-A- 3 637 233
- US-A- 5 609 316

## Beschreibung

In der Chirurgie finden mehr und mehr Operationsmikroskope Anwendung, die infolge ihres hohen Eigengewichtes von Stativen getragen werden müssen. Eine Reihe namhafter Hersteller brachte Stative auf den Markt, die in mechanischer und statischer Hinsicht den Anforderungen der Lastaufnahme des Operationsmikroskops gut entsprechen. Die Anmelderin vertreibt z.B. Stative mit der Bezeichnung OH, die u.a. von Mitaka hergestellt wurden. Ein Beispiel für ein solches Stativ findet sich in der EP 0 628 290 A1 und in der EP 0 476 552 A1. Die meisten der moderneren Stative verfügen über Parallelogrammträger, um die Last der Operationsmikroskope über möglichst grosse Distanzen biege- und verwindungsfrei tragen zu können, so dass die Bewegungsfreiheit und der Aktionsradius der Mikroskope möglichst gross sind. Ein derartiger Aufbau ist in der EP 0 628 290 A1 dargestellt.

Auch die Firma Contraves brachte ein ähnliches Stativ auf den Markt mit zwei getrennten Ausgleichsgewichten, wobei eines am ausgleichskraftübertragenden horizontalen Parallellenker in horizontaler Richtung und das andere an eben diesem in vertikaler Richtung verschiebbar ist. Ein solches Stativ ist auch in der EP 0 476 551 B1 beschrieben.

Einer der Gedanken der Hersteller von herkömmlichen Operationsstativen geht in die Richtung, dass grössere Massivität der Bauteile und höhere Gewichte (Ausgleichsgewichte) gut für die Stabilität des Stativs während seiner Anwendung sind. So waren z.B. unter der Bezeichnung "OH" vertriebene Stative beispielsweise aus relativ massiven Aluminiumgussrohren aufgebaut, die zur integrierten Schwingungsdämpfung zum Teil unterschiedliche Querschnitte mit gestuften Übergängen aufwiesen.

OH-Stative entsprechen, wie bereits erwähnt, der EP 0 628 290 A1 und wurden durch die Anmelderin dieser EP 0 628 290 A1 gemeinsam mit der Anmelderin der vorliegenden Erfindung auf den Markt gebracht.

Um für die Anwender jedoch Verbesserungen zu erzielen wurde eine eigene Entwicklung, mit dem Ziel verbesserter Stativ-Eigenschaften, durchgeführt. Das Ergebnis dieser Entwicklung ist in der WO 97/13997 A1 und der WO 97/20166 A1 beschrieben.

Neu ging die Anmelderin bei der vorliegenden Erfindung auch von der erfindungsgemässen These aus, dass auch leichte Mikroskope eine gute Stabilität haben könnten, sofern sie konstruktiv über verbesserte Bauteile verfügten. Als wesentliche Vorteile gegenüber den bekannten massiven Stativen würden sich daraus eine bessere Transportierbarkeit und damit auch eine universellere Anwendbarkeit ergeben (weniger Probleme mit der Tragfähigkeit des Untergrundes usw.). Andererseits sollte es möglich sein, bei gleichem Gewicht des Mikroskops grössere Aktionsradien rund um das Stativ für die Anwender zu erlauben.

Erfindungsgemäss wurde dabei erkannt, dass die Gewichtsreduktion alleine u. U. nicht ausreichend ist, sofern dabei die Qualität der Dämpfungseigenschaften der wesentlichen Bauteile nicht ausreichend beachtet wird.

Die zur Anwendung gelangenden erfindungsgemässen Träger, die bei Bedarf auch nach wie vor für den Aufbau von Parallelogrammträgern angewendet werden können, sollen möglichst gerade, einfache Bauteile mit grosser Festigkeit sein.

In Erfüllung dieser Aufgaben schuf die Anmelderin ein Stativ, das wenigstens einen Träger aus einem faserverstärkten Kunststoff einsetzt. Dieses Stativ ist in der erwähnten WO 97/20166 A1 beschrieben.

Ausgehend vom Konzept des Ersatzes herkömmlicher paralleler Arme aus Stahl oder Aluminium durch erfindungsgemässe faserverstärkte Kunststoffelemente, insbesondere Rohre, lässt sich Gewicht einsparen bei gleichzeitiger Erhöhung der Festigkeit oder der Aktionsradien. Das Stativ wird daher leichter. Dieser Effekt erhöht sich noch dadurch spürbar, dass das Eigengewicht der Träger ebenso wie das Gewicht der Last, das durch Ausgleichsgewichte kompensiert werden muss, reduziert ist.

Die Einführung faserverstärkter Kunststoffrohre im Mikroskopstativbau ist zwar revolutionär und unterstützt die Lösung der eingangs genannten Aufgaben, jedoch berücksichtigt dieses neue Prinzip noch nicht explizit das Schwingungsverhalten, das an einem Stativ auftreten kann.

In einem anderen Gebiet des Stativbaus, nämlich in der Röntgentechnik, wurde ebenso der Weg der Gewichtsreduktion mittels Fasserverbundstoffen und Kunststoff gegangen, wie in der DE 42 14 858 C1 dargelegt ist. Dort wurde ein C-Bogen als Tragteil aus Kunststoff-Schaum geschaffen, der die Gestalt bestimmt, und von einem faserverstärktem Kunststoff umgeben ist, der die Trageigenschaften übernimmt. Soll dieser bekannte Aufbau ein besonders geringes Gewicht aufweisen, so wird gemäss dieser vorveröffentlichten Lehre verlangt, ein Profil in geschlossener Form aus (nur) faserverstärktem Kunststoff herzustellen.

Die vorliegende Erfindung hingegen erkennt, dass durch diese bekannte Lehre das Ziel nicht optimal erreicht wird. Zwar lässt sich durch moderne Kunststoff-Faser-Verbundaufbauten hohe Stabilität bei guten Schwingungseigenschaften und geringem Gewicht erreichen, jedoch ist dies für den Anwendungsfall in der Mikroskopie, insbesondere in der Operationsmikroskopie nicht genügend, nur "gute" Werte zu erreichen, wenn man die häufig lebensabhängige Wichtigkeit der optimalen Sichtverhältnisse eines Operateurs berücksichtigt.

Beim kleinsten Lageverändern des Mikroskops kommt es unweigerlich zu einer Schwingungserregung am gesamten Aufbau. Aufgabe der vorliegenden Erfindung ist es daher, neben einer weiteren Bauteiloptimierung, Lösungen zu finden, die das Schwingungsverhalten des Stativs positiv beeinflussen, d.h. die Schwingung zu unterbinden oder allfällige Schwingungen optimal zu dämpfen. Dabei sollen niederfrequente Schwingungen, z. B. im Bereich von 0-5 Hz, bekämpft werden.

Der Fachmann weiss, dass solche Aufgaben schwer lösbar sind und dass die Anwendung mathematisch-physikalischer Hilfsmittel und Theorien häufig nicht die erwarteten Resultate bringt. Andererseits sind aber schon geringfügige Verbesserungen erstrebenswert, da diese den Komfort des Anwenders steigern und demzufolge die Operationssicherheit anheben.

Gelöst wird diese Aufgabe erfindungsgemäss durch zwei alternativ oder gemeinsam eingesetzte Massnahmen.
A) Zum einen werden die hochfesten Stativ-Rohre aus Faserverbundstoffen in ihrem Schwingungsverhalten dadurch gedämpft, dass sie innen oder aussen in ihrer Längserstreckung mit einem anderen Trägermaterial verbunden sind, das einen wesentlich unterschiedlichen - in der Regel kleineren - ElastizitätsModul aufweist.
   Erfindungsgemäss soll somit nicht ein einziges Trägermaterial, wie z.B. in der erwähnten DE 42 14 858 C1 und ein formgebendes Material, sondern zwei Trägermaterialien mit gänzlich unterschiedlichen Schwingungseigenschaften eingesetzt werden. In der eben erwähnten DE 42 14 858 C1 wird in Fig.3 zwar auch ein Kombination aus einem Aluminium-Strangpressprofil und einem faserverstärktem Kunststoff angegeben, jedoch zu einem anderen Zweck, in einem anderen Stativ und in einer Art, die dem erfindungsgemässen Ziel nach Optimierung der Gewichts/Schwingungsdämpfungsrelation nicht näher kommt.
   Die vorveröffentlichte Lehre dieser DE 42 14 858 C1 sieht als nicht gewichtsoptimierte Lösung vor, anstelle von Drähten, die der Lagerung von Rollen dienten, das erwähnte Aluminiumstrangprofil in dem faserverstärkten Kunststoff einzubetten. Das dabei gemäss geltender vorbekannter Lehre angewendete Aluminium-Strangpressprofil verfügt über praktisch kein vertikales Widerstandsmoment. Dies ist auch nicht erforderlich, da dieses durch das rohrförmige Kunststoffrohr mit der Faserverstärkung aufgebracht wird. Der Grund, für den Einsatz des Strangpressprofils liegt denn auch nicht in der Optimierung eines Gewicnts/Schwingungsdämpfungsverhaltens, sondern in der verbesserten Halterung von peripheren Drähten für die erwähnte Rollenlagerung.
   Demgegenüber sieht eine erfindungsgemässe Verbindung eine Kombination von rohrförmigen Bauteilen vor, z.B. ein Aluminiumrohrsegment, das aussen oder innen an einem Faserverbundrohr angeklebt ist. Es könnte aber auch - wie bevorzugt - ein Aluminiumrohr sein, auf dem das Faserverbundrohr festhaftend aufgebracht ist, so dass es sich dabei selbst um einen Verbundaufbau handelt (Metall/Kunststoff oder Kunststoff/Metall oder entsprechende Sandwichaufbauten mit einer Wiederholung des einen oder anderen Materials). Aluminium bietet sich dabei aus Gewichtsgründen und wegen seines geringeren Elastizitätsmoduls (E-Moduls) bevorzugt an; die Erfindung ist jedoch nicht auf dieses Material eingeschränkt. Der Fachmann kennt auch andere Materialien, mit denen er Aluminium ersetzen könnte. So könnten z.B. besonders weichlegierte Stähle oder zum Faserverbundmaterial einen unterschiedlichen E-Modul aufweisende Kunststoffe (gegebenenfalls sogar auch faserverstärkt) eingesetzt werden.
   Erst durch die erfindungsgemässe Forderung nach Dämpfungsoptimierung und die Erkenntnis, dass beide Trägermaterialien ein vertikales Widerstandmoment - allerdings mit unterschiedlichen Elastizitätsmodulen - beitragen müssen, führt zur tatsächlichen Trägeroptimierung hinsichtlich der Gewichtsminimierung bei gleichzeitiger Dämpfungsoptimierung.
   Ausführungsformen könnten als Kunststoffe für das hochmodulige wie auch für das niedermodulige Material Thermoplaste, Duroplaste, Thermoset (Epoxidharze) oder eine Mischung daraus einsetzen, wobei als Fasermaterial Carbonfasern, Aramidfasern, Glas- oder Mineralfasern, Polyamidfasern, natürliche Fasern oder Gewebe, oder eine Mischung daraus bevorzugt werden.
   Hinsichtlich der Verbindung zwischen dem hoch- und niedermoduligen Material ist zu erwähnen, dass diese nicht auf eine starre Verbindung - z.B. auf eine Klebeverbindung - eingeschränkt ist; es könnte sich auch um eine lose Steckverbindung handeln, so dass die beiden Rohre - in gewissem Rahmen - gegeneinander axial verschieblich sind.
   Dies kann auch durch einen elastischen Klebstoff realisiert sein. Gegebenenfalls könnte anstelle eines Klebstoffs auch eine zusätzlich dämpfende - z.B. reibende - Zwischenschicht vorgesehen werden.
   Andererseits ist die Erfindung diesbezüglich aber auch nicht auf die Anwendung von Faserverbundmaterial eingeschränkt, sondern umfasst auch Verbundaufbauten aus unterschiedlichen Metallen, wie z.B. Stahl und Aluminium.
B) Zum anderen werden zwischen den hochfesten Teilen und/ oder zwischen dem Stativ und seiner Montage- oder Aufstellfläche an wenigstens einer Stelle dämpfende Materialien angeordnet, wie dies in der erwähnten WO 97/20166 A1 angegeben ist. Dabei wird jedoch nach der Möglichkeit getrachtet, die Leichtgängigkeit allfälliger Gelenke oder Lager zwischen Stativteilen nicht nachteilig zu beeinflussen, da dieses zwar auch dämpfend wirken, dabei aber unerwünschterweise den Bedienkomfort reduzieren könnte.

Die beiden Massnahmen A) und B) können voneinander unabhängig eingesetzt werden; deren Kombination hat sich jedoch als besonders vorteilhaft herausgestellt. Ihr gemeinsamer erfinderischer Aspekt ist die Kombination von Festigkeit mit dämpfender, der Festigkeit scheinbar widersprechender, Eigenschaft.

Gemäss einer Weiterentwicklung der Erfindung kann das Gewicht des Statives bzw. seiner Träger weiter reduziert werden, indem die rohrförmigen Träger vorgespannt werden. Eine solche Vorspannung wirkt hinsichtlich der erfindungsgemässen unterschiedlichen Festigkeitswerte und der unterschiedlichen Elastizitätswerte der beiden Trägermaterialien auf diese auch unterschiedlich, so dass die erfindungsgemässe Dämpfungsoptimierung dadurch nicht ungünstig beeinflusst wird.

Die erfindunsgemässe Idee der Vorspannung ist jedoch nicht eingeschränkt auf Metall-Kunststoffverbundrohre, sondern wäre auch bei einteiligen Stativträgern, wenn es lediglich um die Gewichtsreduktion geht, im Vergleich zum Bekannten vorteilhaft, neu und erfinderisch.

Die Erfindung beschäftigt sich jedoch nicht nur mit den Trägern bzw. rohrförmigen Aufbauten alleine, sondern auch mit dem Stativfuss, der einerseits das Gesamtgewicht des Stativs und seiner Last trägt, hinsichtlich der möglichen Aufnahme von Dämpfungselementen wie Abstellfüssen beachtet wird und andererseits jedoch auch einen bedeutenden Gewichtsfaktor darstellt.

Die Stativfüsse gemäss dem Stand der Technik dienten bei bestimmten Aufbauten durch ihr absichtlich grosses Gewicht der Standfestigkeit. Tigliev verlangt z. B. in seiner US 5 609 316 A, dass der Stativfuss über ein "ausreichendes Gewicht" verfügt, um die "gewünschte Stabilität zu erreichen" (vgl. Spalte 2 Zeile 48-50). Andere moderne Aufbauten, wie z.B. in der WO 97/13997 A1 angegeben, balancieren jedoch den Aufbau so gut, dass es keines schweren Fusses bedarf.

Als weitere Aufgabe soll somit bei hochfester Ausbildung das Gewicht des Fusses reduziert werden.

Diese Aufgabe wird durch einen neuartigen Verbundaufbau des Fusses gelöst, der selbstverständlich auch unabhängig von den oben erwähnten Dämpfungsmerkmalen neu und erfinderisch bei Stativen einsetzbar ist.

Der Aufbau setzt sich im wesentlichen aus einer oberen und einer unteren Platte zusammen, die sandwichartig eine Wabenstruktur aufnimmt, bzw. mit dieser verklebt ist.

Gemäss einer Weiterbildung dieser Erfindung sind die obere und untere Platte an wenigstens einer Stelle zusammengeführt und fest - z.B. mittels Verschraubung- verbunden, so dass scherende Bewegungen zwischen den beiden Platten möglichst vermieden werden. Gleichzeitig ergibt sich dadurch eine besonders gute Haltbarkeit der Wabenkonstruktion zwischen den Platten und ein besonders stabiler, trotzdem jedoch leichter Aufbau.

Bevorzugt sind die Zusammenführungen gleichzeitig als Aufnahmen für Räder oder Abstellfüsse ausgebildet, die bevorzugt absenk- oder hochziehbar sind.

Um das Stativ verfahrbar und andererseits bei Bedarf standfest und gedämpft aufzustellen, sind erfindungsgemäss eine ungerade Anzahl von absenkbaren Abstellfüssen (bevorzugt 5 Stück) vorgesehen, die sich bevorzugt über Dämpfungsmaterial gegen den Boden abstützen.

Die ungerade Anzahl ist von Vorteil hinsichtlich des Kippverhaltens. Als Variante könnte auch ein mehr oder weniger geschlossener Dämpfungsring vorgesehen sein, der wie die Füsse alternativ mit Transporträdern auf den Boden absenkbar ist.

In einer weiteren Ausgestaltung der Erfindung ist den Rädern und/oder Füssen des Stativs eine neuartige Absetzvorrichtung zugeordnet. Diese wird durch einen gemeinsamen Kettentrieb, der mit Zahnrädern kooperiert, von denen jedes mit Steilgewindebolzen verbunden ist, gebildet. Dadurch können mit einer einzigen Handlung gleichzeitig alle Füsse oder Rollen abgesenkt bzw. hochgehoben werden.

Die Erfindung bietet somit für die geometrisch erforderlichen Bauteile besonders geeignete und abgestimmte mechanische Bauteile mit geringerem Gewicht bzw. höherer Festigkeit und verbessertem Schwingungsverhalten. Weitere spezielle Ausbildungen und Varianten dazu sind in den Patentansprüchen beschrieben bzw. unter Schutz gestellt.

Die Verbindung zwischen den faserverstärkten Rohren bzw. Trägern und den übrigen Bauteilen kann mittels eines metallischen Interface erfolgen, das beispielsweise mittels Schrauben oder Splinten oder durch Klebung am jeweiligen Rohr bzw. Träger befestigt werden kann.

Bei der unter A) erwähnten besonderen Ausgestaltung der Erfindung mit statisch und dynamisch optimierten Trägern durch die Ausgestaltung von Trägern des Stativs als Verbundmaterialien mit hochfesten, z.B. faserverstärkten Rohren mit einem hohen Elastizitätsmodul (z.B. 100-210000, insbesondere 150-200000), die mit Metallrohren verbunden sind, werden beim bevorzugten Aufbau gegebenenfalls legierte Aluminiumrohre oder Rohre mit vergleichbarem, nicht hohen E-Modul (ca. 50-80000) verwendet. Als besonderer Vorteil dieses Aufbaus ergibt sich - im Falle einer Faserverstärkung - die Möglichkeit, die Fasern - z.B. Carbonfasern - in Nullage (d.h. parallel zur Rohrachse) zu montieren. Das Metallrohr hält dann gewissermassen über das Kunstharz die Fasern in Position. Die Nullagefasern verbessern einerseits die Biegefestigkeit bzw. Steifigkeit; andererseits wird der Elastizitätsmodul in Torsionsrichtung reduziert.

Im Falle von miteinander verklebten Rohren - insbesondere mit Fasern in Nullage - ergibt sich darüber hinaus eine Versteifung des Metallrohres per se, so dass die Festigkeit sich nicht nur aus der Festigkeit des Metallrohres und der Festigkeit des faserverstärkten Kunststoffes addiert. Die allfällig auftretende Torsion wird durch das Metallrohr - insbesondere Aluminiumrohr - optimal aufgefangen.

Eine weitere, von den obigen Massnahmen auch unabhängig einsetzbare Massnahme wird erfindungsgemäss gesetzt, um allfällig zusätzliche Schwingungsdämpfung im mechanischen Aufbau zu erreichen und derart eine weiter verbesserte Betriebssicherheit zu erreichen:

Gemäss dieser besonderen Ausführung der Erfindung wird, wie schon der WO 97/2016 A1 angegeben, wenigstens eine Schnittstelle zwischen zwei tragenden Bauteilen des Stativs spannungsfrei gehalten. Dies kann im einfachsten Fall dadurch erfolgen, dass die Verbindung zwischen diesen Bauteilen (z.B. eine Schraubverbindung) gelockert wird, so dass die Teile sich zwar nicht voneinander entfernen können, jedoch Vibrationen bzw. Schwingungen - insbesondere im niederfrequenten Bereich - schlecht übertragen werden können.

Zusätzliche Dämpfungseffekte sind erzielbar, wenn, wie unter B) ausgeführt, an den entsprechenden Schnittstellen Dämpfungsmaterialien als Zwischenlager vorgesehen sind.

Als wesentlicher erfindungsgemässer Effekt wird durch diese Massnahme verhindert, dass am Mikroskop auftretende Vibrationen - ausgelöst durch kleine Stösse oder Positionsänderungen - nicht das gesamte Stativ durchlaufen und, gegebenenfalls an der Aufstellfläche (z.B. Fussboden oder Decke) reflektiert, durch das Stativ an ihren Ursprung zurückgelangen können.

Bevorzugte Stellen für die spannungsfreie Trennung sind jene Stellen am Stativ, an denen ein ausbalanciertes Gleichgewicht herrscht und daher kaum Biegespannungen auftreten. Bei einem Ausführungsbeispiel wurde als solche Stelle jene unmittelbar unter dem Hauptlager im Ständer vorgesehen, da das Stativ über dem Hauptlager in einem ausbalancierten Zustand ist, insbesondere wenn es entsprechend der WO 97/13997 A1 aufgebaut ist.

Weitere Bereiche zur Spannungsfreimachung und/oder Einlage von Dämpfungsmaterialien sind gegebenenfalls auch zwischen den Lagerstellen für Räder, Aufstellfüsschen, dem Stativfuss und den übrigen Bauteilen des Stativs.

So kann beispielsweise der zentrale, vertikale Träger auf einem mit dem Fuss - eventuell drehbar - verbundenen Zapfen mit einem elastischen Dämpfungsüberzug aufgesteckt sein.

Auch könnte das vertikale Tragrohr selbst unterbrochen sein, wobei die Unterbrechung durch ein elastisch dämpfendes Zwischenglied überbrückt ist.

Bei der Anwendung von Dämpfungsgliedern im Bereich von Aufstellfüsschen wird bei Anwendung eines grundsätzlichen Zweiwaagenaufbaus gemäss der WO 97/13997 A1 und geeigneter Materialwahl ein zusätzlicher Dämpfungseffekt erzielt:

Ein Pendeln des in einer Horizontalebene schwingenden Stativteils und ein Pendeln des in einer Vertikalebene schwingenden Stativteils kann am senkrechten Stativträger und damit am Fuss zu einem schwingenden Kippmoment in einer vertikalen Ebene bzw. in einer Parallelebene zum Boden führen, das durch die Dämpfungsfüsschen gedämpft wird. Andererseits kann dies aber auch zu einem translatorischen Moment am Fuss - parallel zum Boden - führen. Auch dieses Moment wird durch die bevorzugt angewandten Aufstellfüsschen aus Dämpfungsmaterial - durch Reibungsdämpfung - gedämpft.

Als Dämpfungsmaterial wird erfindungsgemäss bevorzugt ein Material aus Kautschuk oder Polyurethan bzw. Polyetherurethan o.dgl., insbesondere ein geschlossen- oder gemischtzelliger Schaum, angewendet.

Die Eigenschaften des bevorzugten Materials liegen bei etwa folgenden Parametern:
Statischer Elastizitätsmodul 0,2 - 3 N/mm2,
dynamischer Elastizitätsmodul 0,5 - 4 N/mm2
mechanischer Verlustfaktor 0,1 - 0,2
Eigenfrequenz des Materials über 5Hz
jeweils gemessen in Anlehnung an DIN 53513

Als bevorzugtes Material wird beispielhaft Sylomer ® M12, Sylomer ® M25 P14 oder Sylomer ® P12, Sylomer ® P25 P15 für den dynamischen Lastbereich von 0-0,3 N/mm2 gewählt.

Bei Bedarf können dämpfende Materialien auch kombiniert werden. Ebenso liegen im Rahmen der Erfindung Varianten mit bestimmter Formgebung an den Dämpfungsmaterialien. So können z.B. Ausnehmungen, wie Sacklöcher o.dgl. vorgesehen sein, um das Dämpfungsverhalten weiter zu beeinflussen.

Die Erfindung wird anhand von Ausführungsbeispielen mit Hilfe der schematischen Zeichnung näher erläutert. Die Figuren werden zusammenhängend beschrieben. Die Figurenbeschreibung und die Bezugszeichenliste bilden eine Einheit, die sich durch die übrigen Teile der Beschreibung und Ansprüche im Sinne einer vollständigen Offenbarung gegenseitig ergänzen. Gleiche Bezugszeichen bedeuten gleiche Bauteile. Gleiche Bezugszeichen mit unterschiedlichen Indizes bedeuten ähnliche, funktionsgleiche Bauteile. Die Figuren sind nur beispielhaft und nicht zwingend proportional richtig dargestellt. Die Bezugszeichenliste ist kompatibel mit jener der WO 97/13997 A1 und der WO 97/20166 A1. Es zeigen:
Fig.1 eine Schrägansicht eines erfindungsgemässen Stativs mit erfindungsgemässen faserverstärkten Trägern und einem ebensolchen Ständer;
Fig.2 eine Symboldarstellung eines neuartigen Stativs mit einer Zone spannungsfreier Trennung;
Fig.3 eine Symboldarstellung eines erfindungsgemäss angewendeten Stativträgerrohres mit einer Faserorientierung ausserhalb der Nullage;
Fig.4 eine Symboldarstellung eines erfindungsgemässen Verbundstativträgerrohres;
Fig.5 einen Ausschnitt aus einem erfindungsgemässen Stativfuss mit Rollen und absenkbaren Stützfüssen;
Fig.6 einen anderen Ausschnitt dieses Fusses mit abgesenkten Stützfüssen;
Fig.7 einen weiteren Ausschnitt dieses Fusses in Draufsicht;
Fig.8 einen Schnitt durch eine Variante eines erfindungsgemässen Stativfusses;
Fig.9 das Momenten-Schema (Pfeile, die die Schwingung andeuten) an einem symbolischen Ausführungsbeispiel;
Fig.10 einen Schnitt durch einen vorgespannten Träger und
Fig.11 eine Variante dazu.

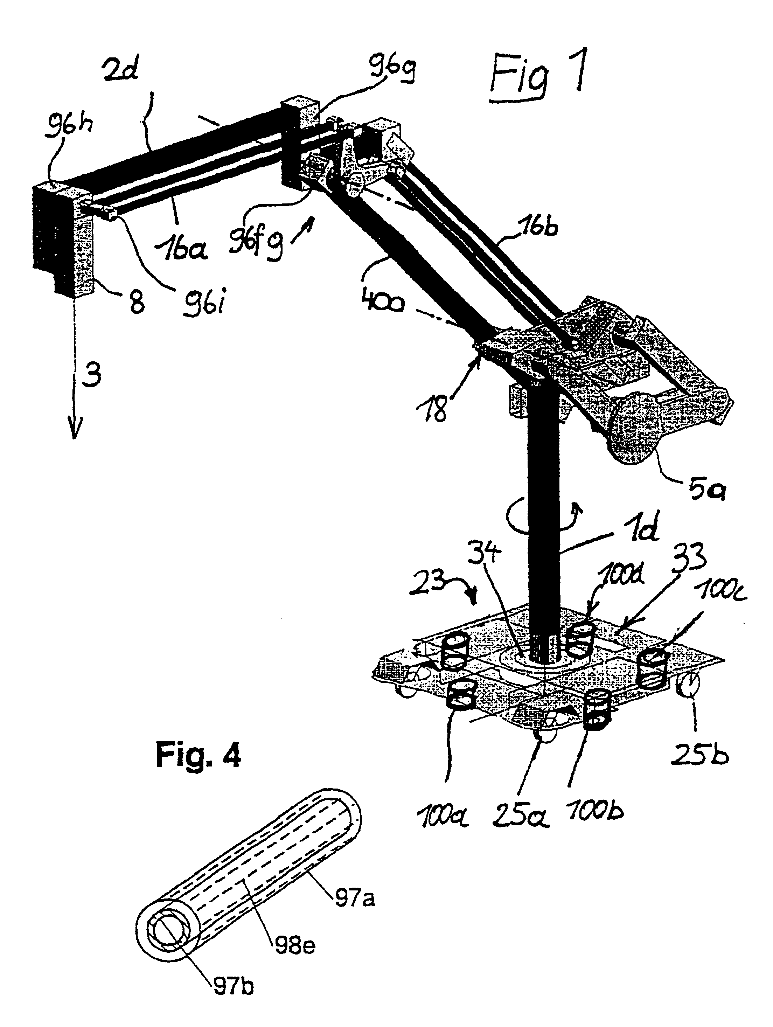

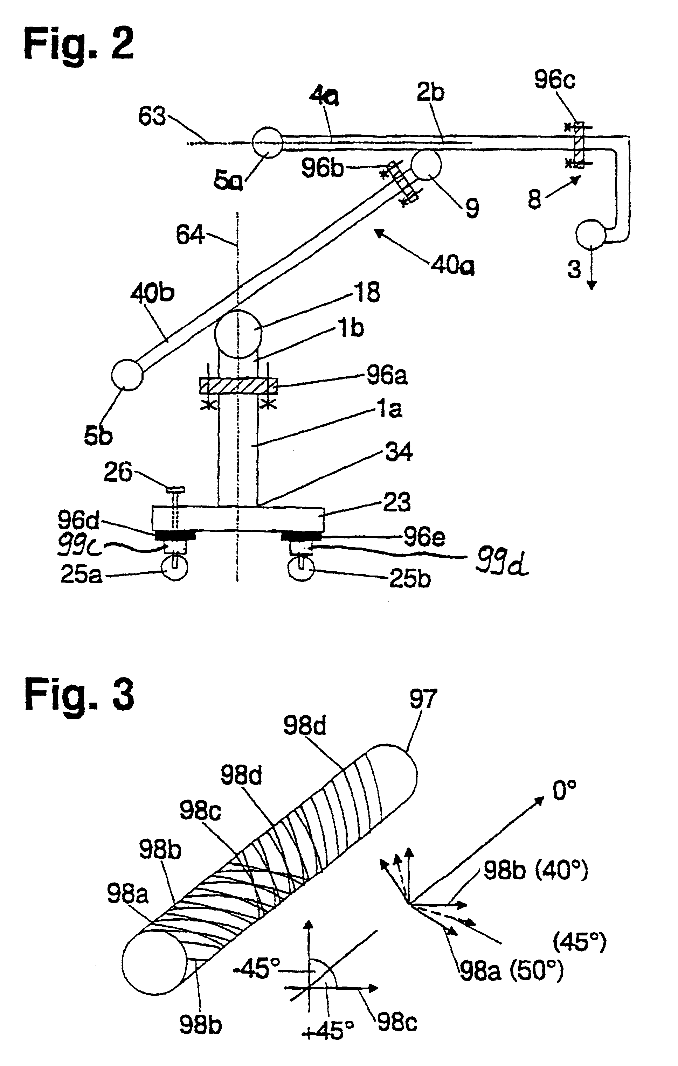

Ein Stativfuss 23 trägt einen Ständer 1, der ein Hauptlager 18 aufnimmt. Gemäss der Variante nach Fig.1 wird der Ständer 1d in einem Drehlager 34 drehbar zum Fuss 23 gehalten. Gemäss Fig.2 ist der Ständer 1 zweigeteilt und mit einem Interface 96a versehen, das - hier im Beispiel - flanschartig ausgebildet ist und den Ständer in zwei Abschnitte 1a und 1b teilt. Es könnte sich aber auch um einen am Ständer 1a befestigten Lagerbock für das Lager 18 handeln, so dass zwischen diesem und dem Ständer 1a das Interface ausgebildet ist. Bei einer Variante wirkt am Interface 96a in vertikaler Richtung bzw. in Richtung der Ständererstreckung keine nennenswerte Spannung, so dass dort Schwingungskräfte nur schlecht übertragen werden. Die dort symbolisch angedeuteten Schrauben sind z.B. nicht vorgespannt. Festigkeitstechnisch spielt dies eine untergeordnete Rolle, da das gesamte Stativ über dem Lager 18 ohnedies ausbalanciert ist, so dass beim Interface 96a praktisch keine nennenswerten Biegekräfte auftreten. Gemäss einer bevorzugten Ausgestaltung ist beim Interface 96a eine dämpfende Zwischenlage angeordnet.

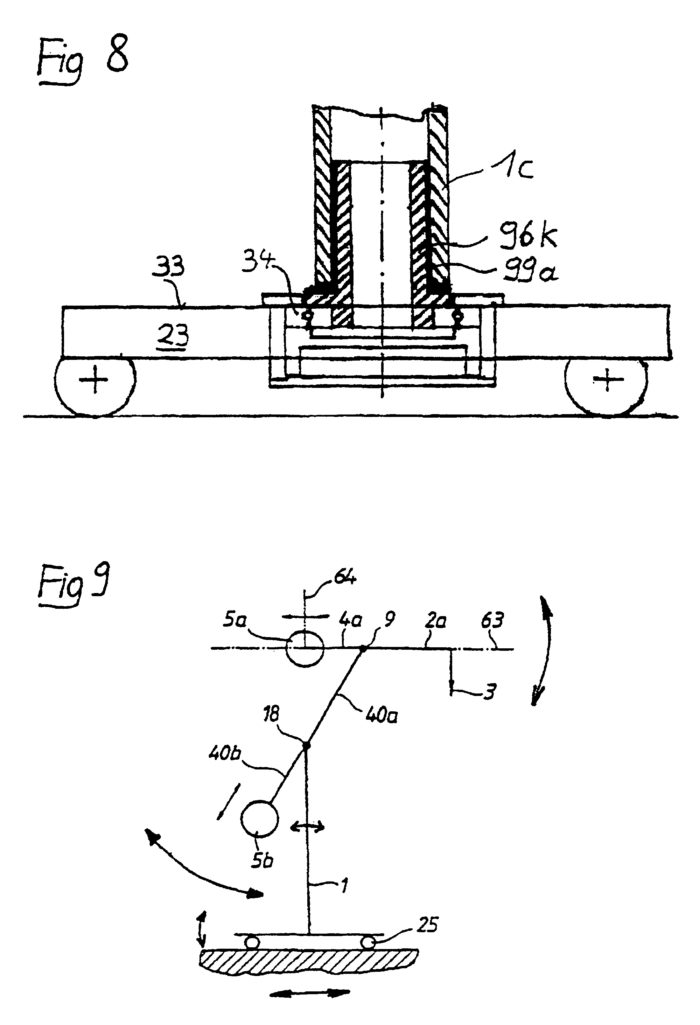

Im Rahmen der Erfindung können aber auch an anderen Stellen vergleichbare Interfaces eingerichtet sein 96b-k. Sie dienen einerseits dazu, die Rohre, die die Träger bilden mit anderen Teilen verbindbar zu machen und andererseits dazu, Schwingungen im System möglichst nicht weiterzuleiten. Insbesondere in Bereichen mit Biegebeanspruchung, z.B. 96a-k, können auch vibrationsdämpfende Zwischenlagen 99 eingebaut sein, deren Aufgabe es ist, mechanische Schwingungen zu vernichten bzw. in Reibungsenergie bzw. Wärme umzuwandeln. Die Fig.8 zeigt z.B. eine besondere Zwischenlage 99a, wie sie den Ständer 1c gegenüber dem Interface 96k aus dem Drehlager 34 des Fusses 23 schwingungsgedämpft lagert. Sowohl Biegekräfteschwingungen als auch Longitudinalschwingungen werden durch diese Zwischenlage 99a gedämpft.

Zusätzlich oder alternativ können, wie in Fig.1 dargestellt, Abstützfüsschen 100 vorgesehen sein, die in der Arbeitsstellung des Stativs dieses gegen den Boden abstützen. Die Abstützfüsschen 100 umfassen schwingungsdämpfende Zwischenlagen 99b, die den Fuss 23 gegenüber dem Boden abstützen (Fig. 5). Solche Zwischenlagen können allerdings auch zwischen den Abstützfüsschen 100 und dem Fuss 23 angeordnet sein.

Die Fig.2 zeigt als Alternative, dass solche Zwischenlagen 99c,d auch zwischen dem Fuss 23 und den Rädern 25a,b angeordnet sein können.

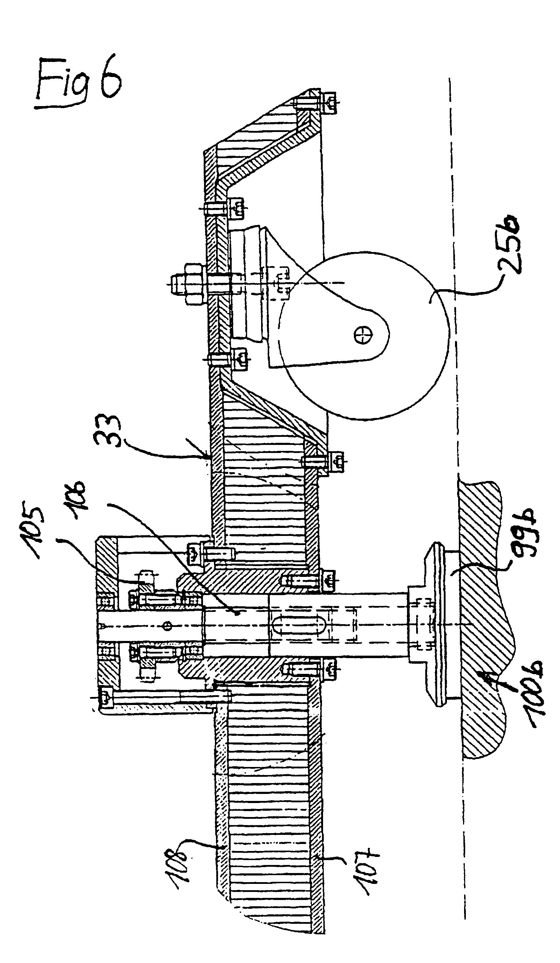

Die Zwischenlagen 99b-d dienen in erster Linie der Dämpfung vertikaler Schwingungen, allerdings auch der Dämpfung von Schwingungen des Ständers 1 um eine horizontale Ebene, da der Fuss 23 diese Schwingungen über seine Hebelarmfunktion in annähernd vertikale Schwingungen an den Zwischenlagen (Puffern) umlenkt. Die Zwischenlagen 99b (Fig. 5 und Fig. 6) fungieren darüber hinaus noch als Reibungsdämpfer zwischen Fuss 23 und Boden für den Fall gewisser Schwingungen des Gesamtsystems aus einer Vertikalebene.

Fig.5-7 geben einen bevorzugten Fuss-Aufbau wieder, bei dem Rollen 25b für den Transportzustand vorgesehen sind und die Abstellfüsschen 100, die über einen Mechanismus abgesenkt werden können. Dieser umfasst bei jedem Füsschen 100 ein Steilgewinde 106, das mit einem Zahnrad 105 verbunden ist, welches über eine Verstellkette 101 gedreht werden kann. Da alle Zahnräder 105 von allen Füsschen 100 von derselben Kette 101 angetrieben werden, kommt es zu einem gemeinsamen Absenken bzw. Anheben der Füsschen 100 beim Wechsel von der Transport- zur Arbeitsposition.

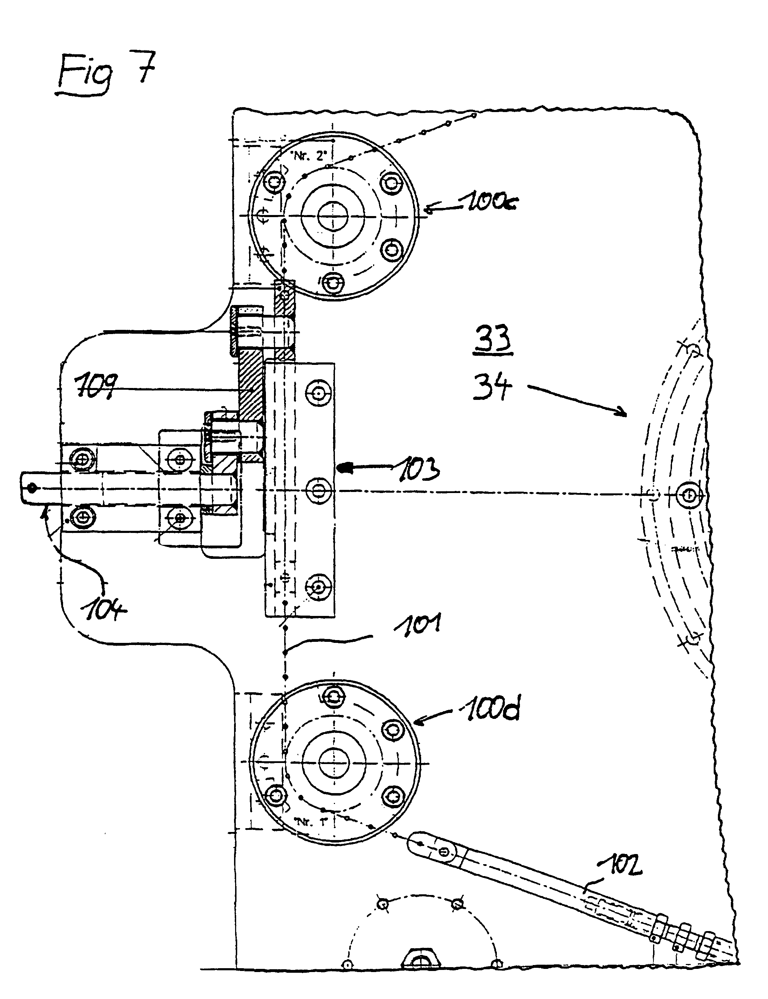

In Fig.7 sieht man noch eine Längenverstellung 102, mit der die Kette 101 gespannt werden kann und einen Stellantrieb 103 mit einer Antriebswelle, über den die Kette 101 angetrieben werden kann. Der Stellantrieb verfügt über einen Exzentertrieb 109, der einerseits ein rasches Verstellen der Füsschen 100 und andererseits eine gute Fixierung derselben in der gewählten Lage ermöglicht.

Der Stativfuss 23 verfügt über einen erfindungsgemässen speziellen Aufbau. Sein Gehäuse 33 verfügt, gemäß der Figuren 5 und 6, über eine untere Platte 107 und über eine obere Platte 108, die im Bereich der Rollen 25 miteinander verbunden sind. Die Verbindung erfolgt über eine topfartige Ausbildung der unteren Platte 107 oder über ein Einsatzstück, das sowohl mit der unteren als auch mit der oberen Platte verschraubt ist. Durch diesen Aufbau werden Zugund Druckkräfte bzw. Biegungen gegenseitig abgestützt. Zur weiteren Versteifung des Fusses 23 ist zwischen den Platten 107 und 108 eine Wabenkonstruktion 110 eingeklebt. Sowohl die Platten als auch die Wabenkonstruktion sind im Sinne des Leichtbaus aus Aluminiumlegierungen aufgebaut.

Etwa zentrisch in der Mitte des Fusses 23 ist eine Ausnehmung 111 vorgesehen (Fig. 5), die das Drehlager 34 für den Ständer 1 aufnimmt.

Die Träger 1,2,4,16,40 sind bevorzugt aus faserverstärktem Kunststoff aufgebaut und dementsprechend besonders leicht, so dass die Ausgleichsgewichte 5 (Fig. 1) ebenso leicht sein können und der Gesamtaufbau gegenüber herkömmlichen Aufbauten gewichtsreduziert ist.

Fig.3 zeigt symbolisch, wie die Fasern 98 im Ausführungsbeispiel orientiert sind. Vier Faserlagen 98a-98d schwanken in einem Winkel von ± 40°-50° zu 0° (Richtung des Rohres 97, das als Träger zum Einsatz kommt). Benachbarte Winkellagen (40°,50°) führen zu einer wirksamen Winkellage von 45° (98c), die für das Erzielen der Biege- bzw. Torsionssteifigkeit Bedeutung hat. Solche geringen Winkeldifferenzen erhöhen jedoch gegenüber einer einlagigen Winkelrichtung (z.B. nur 45°) etwas die Bruchfestigkeit, da sich die benachbarten Fasern offensichtlich gegenseitig die sonst bevorzugte Bruchrichtung entlang der Wickellage sperren.

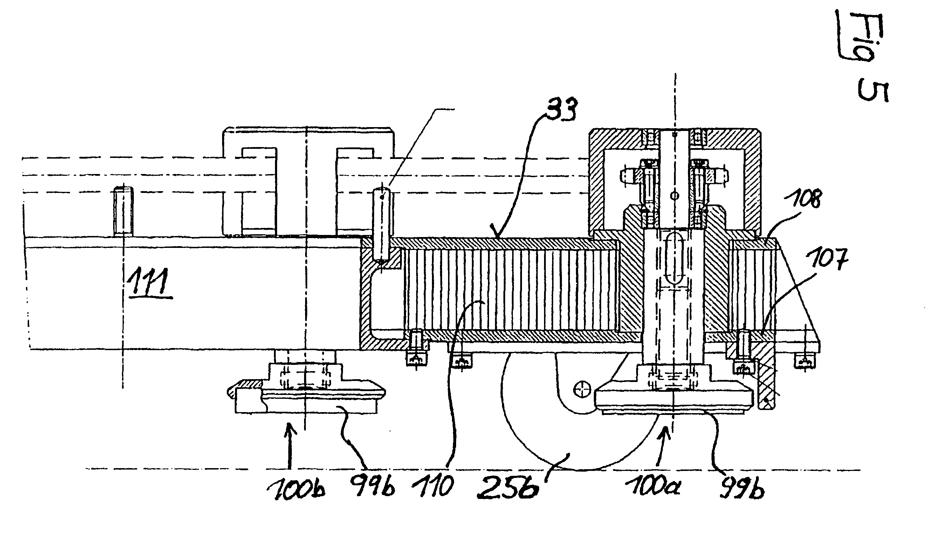

Die Faserorientierung der Fasern 98e gemäss dem Verbundrohr 97a nach Fig. 4 ist in Nullage. Das Rohr 97a ist mit einem Aluminiumrohr 97b oder einem anderen Rohr mit deutlichem Elastizitätsmodulunterschied verbunden. Gegebenenfalls kann das Aluminiumrohr auch an der Aussenseite des Rohres angebracht sein. Verbundrohre mit Sandwichaufbau liegen ebenso im Rahmen der Erfindung. Die Verbindung zwischen den beiden Rohren kann auch schubelastisch sein, so dass geringe axiale Bewegungen zueinander - vorzugsweise reibungsgedämpft - möglich sind.

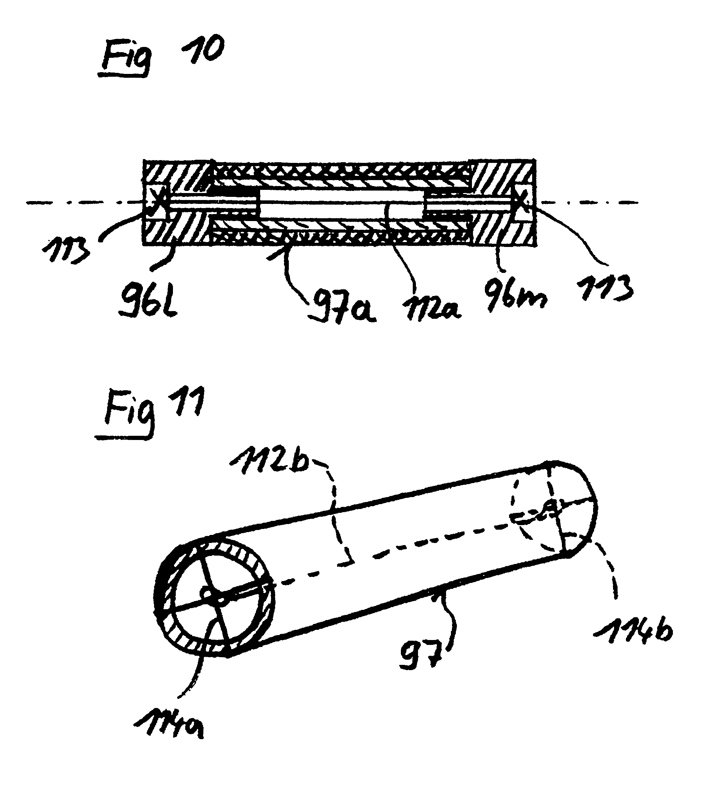

Die besonderen, vorgespannten Varianten gemäss Fig. 10 und 11 haben den Vorteil, bei weiter reduziertem Gewicht hohe Festigkeit aufzuweisen. Die Möglichkeit zur Vorspannung ist sowohl bei horizontalen als auch vertikalen Trägern sinnvoll anwendbar.

Je ein metallisches Interface gemäss 96L bzw. 96m begrenzen gemäss Fig.10 ein Verbundrohr 97a. Axial in Bezug auf das Rohr 97a ist durchgehend eine Spannstange 112a angeordnet, die mittels drehgesicherten Spannschrauben 113 in jedem Interface 96L, 96m abgestützt bzw. vorgespannt ist. Die Wahl der Vorspannung wird bei der Montage z.B. mittels Drehmomentschlüssel durchgeführt.

Bei der Variante gemäß der Fig.11 ist hingegen ein Spannseil 112b vorgesehen, dass an beiden Enden an einem Spanndrahtlager 114a bzw. 114b befestigt ist. Symbolisch dargestellt ist es um das Spanndrahtlager geschlauft. Selbstverständlich kann es aber auch vergleichbar der Fig.10 mittels einer Schrauben-Mutternkonstruktion gehalten sein. Nicht näher dargestellt ist ein Spannmechanismus, der an sich jedoch vielfach bekannt ist, 'zum Spannen von Drähten oder Seilen.

Durch ein entsprechendes Vorspannen der Stange 112a bzw. des Seiles 112b ergibt sich eine überproportionale Erhöhung der Biegesteifigkeit der Rohre 97a bzw. 97, ohne eine nennenswerte Gewichtserhöhung des Mikroskopstativaufbaus. Demzufolge kann bei gleicher Biegesteifigkeit das Gewicht sogar reduziert werden.

Zur weiteren Schwingungsdämpfung kann der Hohlraum im Rohr bzw. Verbundrohr gegebenenfalls auch ausgeschäumt sein.

Die oben beschriebenen unterschiedlichen Massnahmen zur Dämpfung können durch ihre Kombination noch verbessert werden:
Faserverbund/Metallrohre als Träger, gedämpfte oder schwingungsgebrochene Interfaces in der Stativerstreckung und gedämpfte Abstützungen gegenüber dem Boden. Je geringer die Massen dabei sind, umso geringer sind auch die trägen Schwingungsmassen.

### Bezugszeichenliste

- 1a-d: Ständer
- 2,a,b,d: Lastarm, eventuell aus mehreren Stäben aufgebaut; z.B. eine oder mehrere Parallelogrammführungen;
- 3: Last, z.B. ein Operationsmikroskop, könnte aber auch ein beliebiger Bauteil sein, der an einem Stativ zu halten ist, z.B. Roboterarm, Videokamera, Fernrohr o.dgl.
- 4a: Ausgleichsarm, eventuell aus mehreren Stäben aufgebaut; z.B. eine oder mehrere Parallelogrammführungen;
- 5a,b: Ausgleichsgewicht verschiebbar;
- 8: Lastaufhängung, umfasst Vorrichtungen zur Aufnahme eines Mikroskops oder sonstiger Lasten; insbesondere eines Schwenkträgers für ein Mikroskop.
- 9: Schwenkachse (Horizontalschwenkachse) für den Lastarm 2 am und/oder Ausgleichsarm 4, an der diese aus einer horizontalen Ebene 63 schwenken können;
- 16a,b: Zugarm horizontal (a) vertikal (b)
- 18: Schwenkachse (Vertikalschwenkachse), um die das Stativ aus einer vertikalen Ebene 64 schwenken kann.
- 23: Fuss des Stativs
- 25a,b: Räder für Fuss, können starr (nur eine bevorzugte Transportrichtung) oder drehbar befestigt sein; sind bevorzugt fixierbar oder in den Fuss 23 einziehbar, um ein Absetzen des Fusses am Boden zu ermöglichen, wenn keine separaten Abstellfüsschen 100 vorgesehen sind;
- 33: Gehäuse
- 34: Drehlager;
- 40a,b: Schwenkständer, ist der im Ruhezustand senkrechte Bauteil, der das Horizontalschwenklager 9 trägt;
- 63: horizontale Ebene
- 96a-m: Interface, Verbindung zwischen benachbarten Teilen des Stativs, gegebenenfalls mit elastischer, schwingungsdämpfender Zwischenlage 99 z.B. aus einem Elastomer, Polyurethan oder Kautschuk o.dgl. mit hoher Dämpfung;
- 97: faserverstärktes Rohr für Stativträger;
- 97a: Verbundrohr metall/faserverstärktes Rohr;
- 98a-e: Faserlagen aus Carbon o.dgl.;
- 99,a-d: schwingungsdämpfende Zwischenlage;
- 100a-d: Abstützfuss, Stellfüsschen;
- 101: Verstellkette;
- 102: Längenverstellung;
- 103: Stellantrieb;
- 104: Antriebswelle;
- 105: Zahnrad;
- 106: Steilgewindetrieb;
- 107: untere Platte;
- 108: obere Platte;
- 109: Exzenter;
- 110: Wabenkonstruktion;
- 111: Ausnehmung;
- 112: a) Spannstange mit Gewindeenden
- 112: b) Spanndraht
- 113: Spannmuttern
- 114: a,b Spanndrahtlager insbesondere gekreuzt

## Patentansprüche

1. Mikroskopstativ insbesondere für ein Operationsmikroskop mit vertikalen und horizontalen rohrförmigen Trägern (1, 2, 16, 40, 97), **dadurch gekennzeichnet, dass** wenigstens einer der Träger (1, 2, 16, 40, 97) aus zwei rohrförmigen Trägermaterialien mit wesentlich unterschiedlichen Elastizitätsmodulen aufgebaut ist, wobei das eine Trägermaterial konzentrisch das andere umschließt und wobei beide Trägermaterialien zum vertikalen Widerstandsmoment des Trägers beitragen, und/oder dass zwischen hochfesten Stativteilen und benachbarten Stativteilen wenigstens eine Dämpfungsschicht (99) angeordnet ist.

2. Mikroskopstativ nach Anspruch 1, **dadurch gekennzeichnet, dass** eines der Trägermaterialien aus faserverstärktem Kunststoff, z.B. aus Thermoplast, Duroplast, Thermoset (Epoxiharze) oder einer Mischung daraus aufgebaut ist, und dass als Fasermaterial Carbon-, Aramid-, Glas-, Mineral- oder Polyamidfasern oder eine Mischung daraus vorgesehen ist, während das andere Trägermaterial aus Metall mit einem vergleichsweise niedrigen Elastizitätsmodul, z.B. Aluminium, aufgebaut ist.

3. Mikroskopstativ nach Anspruch 2, **dadurch gekennzeichnet, dass** die Fasern nach wenigstens einer der folgenden Wickelmethoden orientiert sind: Filament Winding, Flechtschlauch, Gewebe und Gelege, unidirektional oder in einem Nullwinkel zur Trägererstreckung.

4. Mikroskopstativ nach einem der vorhergehenden Ansprüche, **dadurch gekennzeichnet, dass** die beiden Trägermaterialien starr oder gedämpft schubelastisch miteinander verbunden sind.

5. Mikroskopstativ nach einem der vorhergehenden Ansprüche, **dadurch gekennzeichnet, dass** wenigstens einer der Träger (1,2,16,40,97), insbesondere jener aus faserverstärktem Kunststoff, über wenigstens ein - gegebenenfalls metallisches - Interface (96) verfügt, das ihn mit einem benachbarten Teil verbindet, oder das ihn zweiteilt und verbindet, wobei die Verbindung vorzugsweise spannungsfrei ausgebildet ist.

6. Mikroskopstativ nach einem der vorhergehenden Ansprüche, **dadurch gekennzeichnet, dass** die Dämpfungsschicht (99) wenigstens einem Interface (96) zugeordnet ist, und dass diese Schicht bevorzugt einen gemischtzelligen Schaum, aus einem elastomeren Material, umfasst.

7. Mikroskopstativ nach Anspruch 5 oder 6, **dadurch gekennzeichnet, dass** allfällige Räder (25) und/oder Stellfüsschen (100) des Stativfusses (23) gegenüber diesem oder gegenüber dem Boden mit einer Dämpfungsschicht (99) beabstandet sind.

8. Mikroskopstativ nach einem der vorhergehenden Ansprüche, **dadurch gekennzeichnet, dass** der Stativfuss (23) eine untere Platte (107) und eine obere Platte (108) umfasst, die mittels eingeklebter Wabenkonstruktion (110) voneinander beabstandet sind.

9. Mikroskopstativ nach Anspruch 8, **dadurch gekennzeichnet, dass** die beiden Platten (107,108) an wenigstens einer Stelle starr miteinander, vorzugsweise durch eine Verschraubung, verbunden sind.

10. Mikroskopstativ nach einem der vorhergehenden Ansprüche, **dadurch gekennzeichnet, dass** eine Stellvorrichtung für das Absenken von Stellfüsschen (100) vorgesehen ist, mit der gleichzeitig alle Stellfüsschen abgesenkt werden können.

11. Mikroskopstativ nach Anspruch 10, **dadurch gekennzeichnet, dass** die Stellvorrichtung eine Verstellkette (101), Zahnräder (105) und einen Exzenter (109) umfasst.

12. Mikroskopstativ nach einem der vorhergehenden Ansprüche, **dadurch gekennzeichnet, dass** wenigstens einer der Träger (1,2,16,40,97) in seiner axialen Erstreckung vorgespannt ist.

13. Mikroskopstativ nach Anspruch 12, **dadurch gekennzeichnet, dass** die Vorspannung durch ein zentral geführtes Spannelement (112a,112b) erreicht wird, das gegenüber dem jeweiligen Träger (1,2,16,40,97) auf Dehnung beansprucht ist.

## Claims

1. Microscope stand, especially for a surgical microscope, with vertical and horizontal tubular support elements (1 ,2, 16, 40, 97), **characterized in that** at least one of the support elements (1, 2, 16, 40, 97) is constructed from two tubular support-element materials with significantly different moduli of elasticity, the one support-element material concentrically surrounding the other and both support-element materials contributing to the vertical moment of resistance of the support element, and/or **in that** at least one damping layer (99) is arranged between high-strength stand parts and neighbouring stand parts.

2. Microscope stand according to Claim 1, **characterized in that** one of the support-element materials is constructed from fiber-reinforced plastic, for example from a thermoplastic or thermosetting material (epoxy resins) or a mixture of these, and **in that** carbon, aramid, glass, mineral or polyamide fibres or a mixture of these is provided as the fibre material, while the other support-element material is constructed from metal with a comparatively low modulus of elasticity, for example aluminium.

3. Microscope stand according to Claim 2, **characterized in that** the fibres are oriented according to at least one of the following winding methods: filament winding, woven and nonwoven fabrics, unidirectional or at angle of zero with respect to the extent of the support element.

4. Microscope stand according to one of the preceding claims, **characterized in that** the two support-element materials are connected to each other in a rigid or damped shear-elastic manner.

5. Microscope stand according to one of the preceding claims, **characterized in that** at least one of the support elements (1, 2, 16, 40, 97), especially the one made of fibre-reinforced plastic, has at least one - optionally metallic - interface (96), which connects it to a neighbouring part, or which divides it into two and connects it, the connection preferably being formed in a stress-free manner.

6. Microscope stand according to one of the preceding claims, **characterized in that** the damping layer (99) is assigned to at least one interface (96), and **in that** this layer preferably comprises a mixed-cell foam, made of elastomeric material.

7. Microscope stand according to Claim 5 or 6, **characterized in that** any wheels (25) and/or adjusting feet (100) of the stand foot (23) are spaced apart from the latter or from the base by a damping layer (99).

8. Microscope stand according to one of the preceding claims, **characterized in that** the stand foot (23) comprises a lower plate (107) and an upper plate (108), which are spaced apart from each other by means of an adhesively bonded-in honeycomb structure (110).

9. Microscope stand according to Claim 8, **characterized in that** the two plates (107, 108) are rigidly connected to each other at at least one point, preferably by a screwed connection.

10. Microscope stand according to one of the preceding claims, **characterized in that** an adjusting device for the lowering of adjusting feet (100) is provided, with which all the adjusting feet can be lowered at the same time.

11. Microscope stand according to Claim 10, **characterized in that** the adjusting device comprises an adjusting chain (101), gear wheels (105) and an eccentric (109).

12. Microscope stand according to one of the preceding claims, **characterized in that** at least one of the support elements (1, 2, 16, 40, 97) is prestressed in its axial extent.

13. Microscope stand according to Claim 12, **characterized in that** the prestressing is achieved by a centrally guided stressing element (112a, 112b), which is subjected to tensile stress with respect to the respective support element (1, 2, 16, 40, 97).

## Revendications

1. Statif pour microscope, en particulier pour microscope d'opération, muni de bras tubulaires verticaux et horizontaux (1, 2, 16, 40, 97), **caractérisé en ce qu'**au moins l'un des bras (1, 2, 16, 40, 97) est constitué de deux matériaux porteurs tubulaires présentant des modules d'élasticité nettement différents, l'un des matériaux porteurs enfermant l'autre de manière concentrique et les deux matériaux porteurs contribuant au moment résistant vertical du bras, et/ou **en ce qu'**au moins une couche amortissante (99) est disposée entre des parties à haute résistance du statif et les parties du statif adjacentes.

2. Statif pour microscope selon la revendication 1, **caractérisé en ce que** l'un des matériaux porteurs est à base d'un matériau synthétique renforcé de fibres, par exemple un thermoplastique, un thermodurcissable, une résine époxy ou un mélange de ceux-ci, et **en ce que** les fibres prévues sont des fibres de carbone, d'aramide, de verre, minérales ou polyamides, ou un mélange de celles-ci, alors que l'autre matériau porteur est à base d'un métal présentant un module d'élasticité comparativement bas, par exemple l'aluminium.

3. Statif pour microscope selon la revendication 2, **caractérisé en ce que** les fibres sont orientées au moins selon l'une des méthodes d'enroulement suivantes : enroulement filamentaire, gaine tressée, tissu de fibres, unidirectionnel ou avec un angle zéro par rapport à l'extension du bras.

4. Statif pour microscope selon l'une des revendications précédentes, **caractérisé en ce que** les deux matériaux porteurs sont reliés ensemble de manière rigide ou de manière amortie permettant une élasticité de cisaillement.

5. Statif pour microscope selon l'une des revendications précédentes, **caractérisé en ce qu'**au moins l'un des bras (1, 2, 16, 40, 97), en particulier celui en matériau synthétique renforcé de fibres, dispose au moins d'une interface - le cas échéant métallique - (96) le reliant à une partie adjacente, ou le divisant en deux et reliant les deux parties, la liaison étant de préférence conçue sans contraintes.

6. Statif pour microscope selon l'une des revendications précédentes, **caractérisé en ce que** la couche amortissante (99) est affectée au moins à une interface (96) et **en ce que** cette couche comprend de préférence une mousse à alvéoles ouvertes et fermées en un matériau élastomère.

7. Statif pour microscope selon la revendication 5 ou 6, **caractérisé en ce que** les éventuelles roues (25) et/ou pieds d'appui (100) de la base du statif (23) sont séparés de celle-ci ou du sol par une couche amortissante (99).

8. Statif pour microscope selon l'une des revendications précédentes, **caractérisé en ce que** la base du statif (23) comprend une plaque inférieure (107) et une plaque supérieure (108) séparées l'une de l'autre par une structure collée en nid d'abeille (110).

9. Statif pour microscope selon la revendication 8, **caractérisé en ce que** les deux plaques (107, 108) sont reliées de manière rigide en au moins un point, de préférence par vissage.

10. Statif pour microscope selon l'une des revendications précédentes, **caractérisé en ce qu'**un dispositif de commande est prévu pour la descente de pieds d'appui (100), permettant la descente simultanée de tous les pieds d'appui.

11. Statif pour microscope selon la revendication 10, **caractérisé en ce que** le dispositif de commande comprend une chaîne de commande (101), des pignons (105) et un excentrique (109).

12. Statif pour microscope selon l'une des revendications précédentes, **caractérisé en ce qu'**au moins un des bras (1, 2, 16, 40, 97) est précontraint suivant son extension axiale.

13. Statif pour microscope selon la revendication 12, **caractérisé en ce que** la précontrainte est obtenue à l'aide d'un élément tendeur disposé de manière centrale (112a, 112b) sollicité en extension par rapport au bras concerné (1, 2, 16, 40, 97).
